# EUROPEAN PATENT SPECIFICATION

(11) **EP 3 312 602 B1**
(45) Date of publication and mention of the grant of the patent: **01.05.2019**
(21) Application number: 17195898.6
(22) Date of filing: 11.10.2017
(51) Int. Cl.: G01N 29/02, G01N 29/024, G01N 29/22, G01N 29/24, G01N 29/34, G01N 33/14, G01N 21/43

(54) **DEVICE FOR MEASURING THE PHYSICAL PARAMETERS OF A LIQUID**
VORRICHTUNG ZUR MESSUNG DER PHYSIKALISCHEN PARAMETER EINER FLÜSSIGKEIT
DISPOSITIF POUR MESURER LES PARAMÈTRES PHYSIQUES D'UN LIQUIDE

(30) Priority: 21.10.2016 IT 201600105993
(43) Date of publication of application: 25.04.2018
(73) Proprietor: Maselli Misure S.P.A., 20121 Milano (IT)
(72) Inventor: MASELLI, Andrea, 43126 PARMA (IT); VISENTINI, Giovanni, 43014 MEDESANO (PR) (IT)
(74) Representative: Corradini, Corrado

(56) References cited:
- US-A- 4 918 979
- US-A1- 2014 307 756

## Description

### TECHNICAL FIELD

The present invention relates to a device for the determination of physical parameters of a liquid, for example a ternary liquid solution.

In particular, the invention relates to a device for the continuous and in-line determination of physical parameters of an alcoholic beverage consisting of water, sugars and alcohol, such as for example beer.

### EXISTING TECHNIQUE

As is known, in the industrial and craft processes for producing liquid solutions, for example ternary solutions such as alcoholic beverages, for example beer, it is fundamental to know the concentration of sugars and alcohol in the beverage in all steps of the process itself.

Devices are known for the determination of the sugar concentration in the alcoholic beverages which comprise a refractometer configured for determining the refractive index of the alcoholic beverage itself. Indeed, the refractive index of an alcoholic beverage is the function of the concentration of sugars of the beverage itself. However, the refractive index of the beverage is also a function of the alcohol content in the beverage.

Moreover, devices are known for the determination of the sugar concentration in alcoholic beverages, the device being provided with a sonic unit for the determination of the speed-of-sound propagation in the beverage itself. Indeed, the speed-of-sound propagation in the beverage is a function of the concentration of sugars in the beverage itself. However, the speed-of-sound propagation in the beverage is also a function of the alcohol content in the beverage.

It is therefore common practice to arrange refractometers and sonic units associated with the pipes of a production plant of the alcoholic beverage for the in-line measurement of the refractive index and of the speed-of-sound propagation in the beverage. The concentration of sugars and the alcohol content in the beverage itself may be determined from this information, through convenient interpolations made by means of tables or calculation software.

Known alcoholic beverage production plants normally comprise both refractometers and sonic units connected in various points of the pipes of the plant itself, for example the refractometers are connected to the plant in different points with respect to the sonic units.

The increased number of sensors required obviously affects the installation and maintenance cost of the plant.

Moreover, the determination of the alcohol content in the beverage is given by the correlation of different measurements taken in different points of the line and therefore is subject to an increased level of uncertainty due to contaminations or turbulences in the beverage which may occur during the flow of the beverage itself between the different sensors.

In particular, the beverage may have different physical parameters (for example, temperature) and concentrations along the ducts of the plant. Accordingly, measurements taken in different points of the plant give rise to values which, if correlated with one another, allow the determination of highly uncertain values.

It is an object of the present invention to overcome the mentioned drawbacks of the known technique, within the scope of a simple, rational and affordable solution.

Such objects are achieved by the features of the invention indicated in the independent claim. The dependent claims outline preferred and/or particularly advantageous aspects of the invention.

### DESCRIPTION OF THE INVENTION

One embodiment of the invention makes available a device for the measurement of physical parameters of a liquid, comprising a casing inside of which there are arranged: a refractometer configured for measuring a value of refractive index of the liquid, a sonic unit configured for measuring a value of sound propagation speed in the liquid.

According to the invention, the casing comprises an operative surface adapted to be in contact with the liquid, and the refractometer and the sonic unit are associated with said operative surface of the casing.

Due to such a solution, a single device is made available for the measurement of different physical parameters of the liquid, for example an alcoholic beverage, in particular physical parameters correlated with the concentration of the components of the liquid so as to contain the costs of installing, managing and servicing the liquid production plant.

Moreover, the measuring of the different physical parameters may be made in the same point of the plant, thus decreasing the level of uncertainty of the individual measurements and above all, the uncertainty of the values correlated therewith, such as for example, the concentration of the components of the liquid.

Thereby, the measuring in the same point of the ducts of several parameters also allows an indication on the validity of the measurements themselves to be provided. Indeed, it may be established from the comparison between the values measured of the parameters, if one of them is the result of a false reading due for example, to a malfunctioning of the relative sensor. According to one aspect of the invention, the refractometer may comprise a prism which projects from the operative surface of the casing, and the sonic unit may comprise an emitter and a receiver of an acoustic signal which project from the operative surface of the casing.

Due to such solution, the device is compact and easy to install. Thereby, the measurements also are taken in the same point of the plant, thus reducing errors and uncertainties due to turbulences in the flow of the liquid. According to another aspect of the invention, the prism of the refractometer may be substantially interposed between the emitter and the receiver of the sonic unit.

Due to such solution, the device is compact and easy to install. Thereby, the measurements also are taken on the same volume of liquid interposed between the emitter, the receiver and the prism, thus reducing errors and uncertainties due to turbulences in the flow of the liquid.

According to another aspect of the invention, the device may comprise a temperature sensor associated with the operative surface of the casing.

Due to such solution, the device allows the number of physical parameters monitored to be increased and also allows the measurements taken at the same temperature to be compared with one another, while compensating them at a fixed reference temperature.

According to a further aspect of the invention, the device may comprise an electronic control unit configured for: receiving, a first signal from the refractometer, representative of the refractive index of the liquid; receiving, from the sonic unit, a second signal representative of the sound propagation speed in the liquid; determining the value of the concentration of at least one first component of the liquid depending on the first and second signal.

Due to such solution, the values of the physical parameters measured may be used to obtain further information on the liquid, for example the value of the concentration of sugars in an alcoholic beverage.

The electronic control unit may also be configured for determining the value of the concentration of a second component of the liquid depending on the first and second signal.

Due to such solution, the value of a second component of the liquid may be determined, for example the concentration of alcohol of a beverage, by basing such determination on the measurement of two different physical parameters, but measured at the same point of the plant, thus reducing the level of uncertainty of the measurement.

According to another aspect of the invention, the device may comprise a displaying element associated with the casing and configured for displaying the value of the concentration of the at least one component of the liquid. Thereby, an operator may be immediately and constantly updated on the measured parameters of the liquid.

According to another aspect of the invention, the device may comprise a powering element contained in the casing and configured for powering simultaneously the refractometer and the acoustic unit.

Due to such solution, the device is compact and self-sufficient in terms of power.

According to a further aspect of the invention, the device may comprise means for fixing the casing to a duct of a plant for the production of the liquid. Thereby, the device may be easily installed in a production plant of the liquid.

### BRIEF DESCRIPTION OF THE DRAWINGS

Further features and advantages of the invention will be more apparent after reading the following description provided by way of a non-limiting example, with the aid of the accompanying drawings.

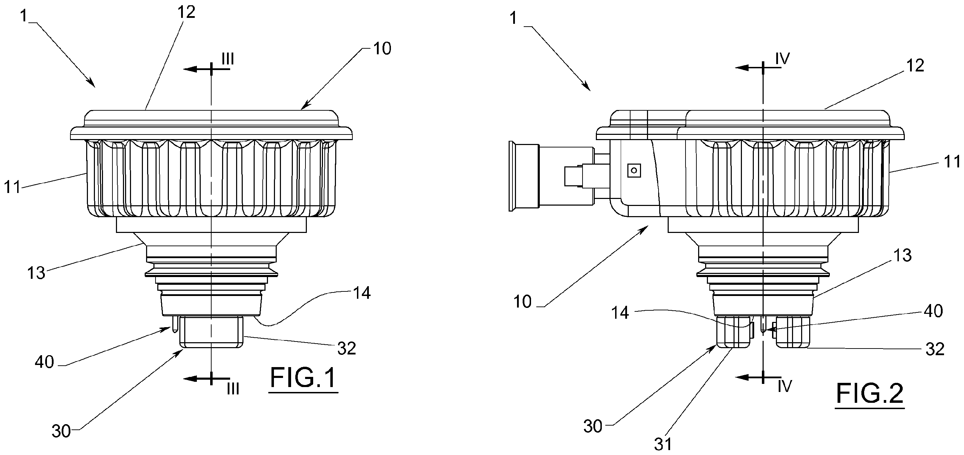

Figure 1 is a front view of the device according to the invention.
Figure 2 is a side view of the device according to the invention.

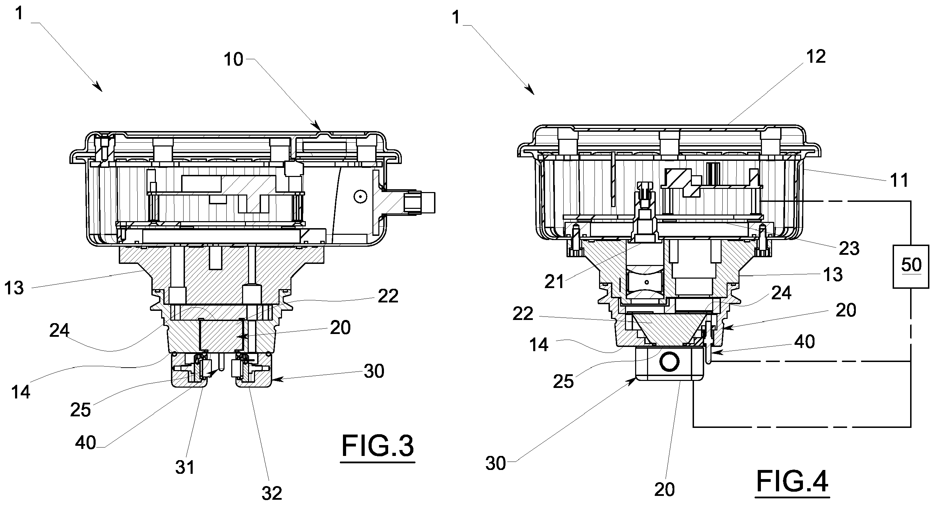

Figure 3 is section III-III of figure 1.
Figure 4 is section IV-IV of figure 2.

### IMPROVED METHOD FOR IMPLEMENTING THE INVENTION

With particular reference to such drawings, indicated as a whole with 1 is a device for measuring the physical parameters of a liquid, for example a ternary solution. In particular, device 1 may be configured for determining the concentration of sugars and/or alcohol in an alcoholic beverage, for example beer.

The device 1 is configured for being installed in a production plant (industrial or small-scale) of the beverage.

Preferably, the device 1 is adapted to be installed on a duct or piping of the plant so as to determine the concentration of sugars and/or alcohol without taking a sample of beverage. In practice, the device 1 is configured for determining the concentration of sugars and/or alcohol in a volume of beverage that flows in the duct close to or in contact with a portion of the device 1 itself, moreover it is configured for performing the determination in continuous manner.

The device 1 comprises a casing 10, for example irregular in shape and provided with a container body 11 closed at the top by a cover 12 defining a wall of the casing 10 substantially opposite to the bottom wall of the container body 11.

In practice, the container body 11 and the cover 12 define a compartment for receiving means for determining the concentration of sugars and/or alcohol in the beverage, as will be described better below.

The container body 11 is for example, tub-shaped and comprises a bottom wall bordered by a side wall which free edge delimits an opening closed by the cover 12.

The container body 11 is provided with a projecting shank 13 adapted to be at least partially inserted in the duct, and is provided with an end constrained to the bottom wall of the container body and an opposite free end adapted to be arranged in the duct.

In particular, the free end of the projecting shank 13 is provided with an operative surface 14 adapted to be placed in contact with the beverage to be analysed, for example it is adapted to be immersed in the beverage. Advantageously, the projecting shank 13 comprises a side wall provided with means for fixing the device 1 to the duct, for example a flange fixed to the duct. In the embodiment shown in the drawings, the fixing means comprise profiles projecting radially from the side wall of the projecting shank 13 adapted to obtain a sealed fixing, for example a bayonet fixing or by threading with respective profiles or grooves made in the flange.

The projecting shank 13 may be made in a single piece with the container body 11 or it may be made as a separate body with respect to the container body and be fixed sealed thereto in a dismountable manner, for example by means of a fixing flange that carries threaded members.

Moreover, the device 1 comprises connectors associated with the side wall of the container body 11 of the casing 10, which allow the connection to external devices such as for example, a computer and/or an external powering source.

According to other embodiments not shown, entirely by way of an option the device 1 could comprise control means (for example, for turning on or turning off the device 1 itself) which may comprise one or more control buttons associated with the cover 12.

Moreover, according to other embodiments not shown, entirely by way of option the device 1 could comprise elements for displaying the concentration of sugars and/or alcohol in the beverage, for example a display associated with the cover 12.

A refractometer 20 configured for measuring the refractive index of the beverage is housed in the casing 10.

The refractometer 20 comprises a light source 21, a prism 22 and an optical sensor 23 adapted to receive a reflective light beam.

The light source 21, for example an LED, is housed in the casing 10 and is configured for emitting a light beam directed towards the prism 22 arranged at the free end of the projecting shank 13.

The refractometer 20 may comprise one or more lenses interposed between the light source 21 and the prism 22 for directing the light beam towards the prism 22 itself.

The prism 22, which is made of optically transparent material with known refractive index nP, is arranged at the free end of the projecting shank 13 and projects onto the operative surface 14 of the casing 10, for example at a central portion of the operative surface 14 itself.

In particular, the prism 22 comprises a first surface 24 inside the casing 10 and facing towards the light source 21, and a second surface 25 outside the casing 10 and adapted in use to be in contact with the beverage, for example the second surface 25 of the prism 22 is placed flush with the operative surface 14 of the casing 10.

The optical sensor 23 is arranged inside the casing 10 in position such as to receive a reflective light beam from the prism 22, for example it is beside the light source 21.

Preferably, the optical sensor 23 is a CCD optical sensor consisting of a series of 2048 pixels placed at a distance of 13 microns from one another. The optical sensor 23 is operatively connected to an electronic control unit 50 and is configured for sending a first signal A representative of the refractive index measured to the electronic control unit 50 itself.

In practice, the light source 21 emits a light beam towards the prism 22. The light beam is composed of a plurality of light rays which meet the interface between the second surface 25 of the prism 22 and the beverage with different angles of incidence α. The rays with an angle of incidence α greater than a limit value β are reflected by the interface between the second surface 25 of the prism 22 and the beverage, while rays with an angle of incidence α less than the limit value β are refracted and dispersed into the beverage.

Since the limit value β of the angle of incidence is characteristic of the interface between the prism 22 and the beverage and in particular, of the refractive indexes of the prism nP and of the beverage nB, by measuring the value of the limit angle, the value of the refractive index of the beverage nB may be measured according to the following relation: nB=nP*sinβ. Operatively, the part of the rays of the emitted light beam reflected by the interface between the second surface 25 of the prism 22 and the beverage form a reflective light beam directed towards the optical sensor 23, while the part of the rays of the emitted light beam refracted into the beverage is dispersed and accordingly, only a portion of the optical sensor 23 is illuminated by the reflective light beam, the remaining part remains in darkness. The position of the borderline between the illuminated part of the sensor and the part in darkness of the sensor is a function of the limit value β of the angle of incidence.

The device 1 also comprises an acoustic unit 30 configured for measuring the speed-of-sound propagation Vs in the beverage.

The acoustic unit 30 comprises an emitter 31 and a receiver 32 associated with the casing 10 so as to be, in use, immersed in the beverage.

In particular, in the embodiment shown, the emitter 31 and the receiver 32 are fixed to the projecting shank 13 of the casing 10, for example to the free end of the projecting shank 13.

In greater detail, the emitter 31 and the receiver 32 project in axial direction from the free end of the projecting shank 13, for example from the operative surface 14 of the casing 10.

Moreover, preferably the emitter 31 and the receiver 32 are fixed to the operative surface 14 of the casing 10 at a peripheral portion thereof, and are arranged spaced apart from each other and in diametrically opposite positions with respect to the second surface 25 of the prism 22.

In practice, the emitter 31 and the receiver 32 of the sonic unit 30 are separated from each other substantially by the second surface 25 of the prism 22 of the refractometer 20.

The emitter 31 is configured for emitting a sound pulse signal, for example an ultrasound pulse signal, directed towards the receiver 32 through the beverage.

The sonic unit 30 is also provided with a control hardware operatively connected with the emitter 31 and the receiver 32, and is configured for: measuring the delay time θ between the emission of the sound pulse by the emitter 31 and the receipt of the sound pulse by the receiver 32; determining the speed-of-sound propagation in the beverage as a function of the delay time θ.

The sonic unit 30 is also operatively connected (for example, by means of the control hardware) to the electronic control unit 50 and is configured for sending a second signal B representative of the propagation speed of the sound pulse in the beverage to the electronic control unit 50 itself.

According to the preferred embodiment, the control hardware of the sonic unit is comprised in the electronic control unit 50 of the device 1. According to other embodiments, the electronic control unit 50 and the control hardware of the sonic unit 30 may be separate. The device 1 also comprises a temperature sensor 40 configured for determining the temperature T of the beverage.

The temperature sensor 40 is advantageously associated with the casing 10, for example it projects from the free end of the projecting shank 13.

For example, the temperature sensor 40 projects in axial direction of the operative surface 14 of the casing 10, for example in a peripheral portion thereof.

The temperature sensor 40 is operatively connected to the electronic control unit 50 and is configured for sending a third signal C representative of the temperature of the beverage to the electronic control unit 50 itself.

In practice, the emitter 31, the receiver 32 and the prism 22 (and the temperature sensor 40) are associated with the casing 10 so as to define an imaginary volume interposed therebetween in which there is collected a portion of quantity of beverage from which the refractive index nB, the speed-of-sound propagation and the temperature are measured.

The device 1 also comprises means for powering the refractometer 20, the sonic unit 30 of the temperature sensor 40 and the electronic control unit 50. For example, the powering means may be inside the casing 10, for example one or more batteries, or outside the casing, for example connected to the casing by means of external connectors associated with the side wall of the container body 11.

According to a preferred embodiment, the electronic control unit 50 is housed in the casing 10 and is configured for receiving, from the refractometer 20, the first signal A representative of the refractive index of the beverage, and is configured for receiving, from the sonic unit, the second signal representative of the speed-of-sound propagation in the beverage; determining the value of the concentration of sugars as a function of at least one between the first and the second signal A and B.

The electronic control unit 50 is also configured for determining the alcohol content in the beverage as a function of the first and the second signal A and B.

In particular, the electronic control unit 50 is configured for determining a first value of the concentration of sugars ZA, for example expressed in Brix, based on the first signal A; determining a second value of the concentration of sugars ZB, for example expressed in Brix, based on the second signal B; calculating the difference Δ between the second and the first value of the concentration of sugars Δ=(ZB-ZA); determining the alcohol content in the beverage X as a function of the difference Δ between the second and the first value of the concentration of sugars ZB, ZA.

Advantageously, the electronic control unit 50 may be configured for receiving the third temperature signal C from the temperature sensor 40; verifying if the first signal A and the second signal B are representative of physical parameters of the beverage measured at the same temperature, compensating them to the value that they would have at a fixed reference temperature TO if the physical parameters were measured at the same temperature.

According to another embodiment not shown, the device 1 might not have the control unit and could comprise solely means for sending the signals A, B, C, representative of the values measured of the physical parameters of the beverage, outside the device itself, for example towards a computer connected remotely with the device 1 itself, for example with a cable connection through connectors associated with the casing 10.

In this case, the device 1 could in any case comprise a single electronic board located inside the casing 10 and configured for acting as hardware both for the refractometer 20 and for the sonic unit 30.

## Claims

1. Device (1) for measuring the physical parameters of a liquid, comprising a casing (10), inside of which there is arranged:
• a refractometer (20) configured for measuring a value of refractive index of the liquid, and
• a sonic unit (30) configured for measuring a value of sound propagation speed in the liquid,
wherein the casing (10) comprises an operative surface (14) adapted for being in contact with the liquid and wherein the refractometer (20) and the sonic unit (30) are associated with said operative surface (14) of the casing (10),
wherein:
• the refractometer (20) comprises a prism (22) projecting from the operative surface (14) of the casing (10); and
• the sonic unit (30) comprises an emitter (31) and a receiver (32) of an acoustic signal, which project from the operative surface (14) of the casing (10),
wherein the prism (22) comprises a first surface (24) inside the casing (10) and a second surface (25) outside the casing (10) and adapted for being in contact with the liquid, **characterised in that** the emitter (31) and the receiver (32) of the sonic unit (30) are separated from each other and the second surface (25) of the prism (22) of the refractometer (20) is situated between the emitter (31) and the receiver (32) of the sonic unit (30).

2. Device (1) according to claim 1, comprising a temperature sensor (40) projecting in axial direction from the operative surface (14) of the casing (10).

3. Device (1) according to any preceding claim, comprising an electronic control unit (50) configured for:
• receiving from the refractometer (20) a first signal representative of the refractive index of the liquid;
• receiving from the sonic unit (30) a second signal representative of the sound propagation speed in the liquid;
• determining the value of the concentration of at least a first component of the liquid depending on the first and second signal.

4. Device (1) according to claim 3, wherein the electronic control unit (50) is configured for determining the value of the concentration of a second component of the liquid depending on the first and second signal.

5. Device (1) according to any one of claims 3 or 4, comprising a displaying element configured for displaying the value of the concentration of the at least one component of the liquid.

6. Device (1) according to any preceding claim, comprising a powering element contained in the casing (10) and configured for powering simultaneously the refractometer (20) and the acoustic unit (30).

7. Device (1) according to any preceding claim, provided with means (13) for fixing the casing (10) to a conveying conduit for the liquid.

## Patentansprüche

1. Vorrichtung (1) zum Messen der physikalischen Parameter einer Flüssigkeit, ein Gehäuse (10) umfassend, in dessen Innerem Folgendes angeordnet ist:
• ein Refraktometer (20), das dafür konfiguriert ist, einen Wert des Brechungsindex der Flüssigkeit zu messen, und
• eine Akustikeinheit (30), die dafür konfiguriert ist, einen Wert der Schallausbreitungsgeschwindigkeit in der Flüssigkeit zu messen,
wobei das Gehäuse (10) eine operative Oberfläche (14) umfasst, die dafür eingerichtet ist, in Kontakt mit der Flüssigkeit zu sein, und wobei das Refraktometer (20) und die Akustikeinheit (30) mit der operativen Oberfläche (14) des Gehäuses (10) verbunden sind,
wobei:
• das Refraktometer (20) ein Prisma (22) umfasst, das aus der operativen Oberfläche (14) des Gehäuses (10) hervorsteht, und
• die Akustikeinheit (30) einen Emitter (31) und einen Empfänger (32) für ein akustisches Signal umfasst, die aus der operativen Oberfläche (14) des Gehäuses (10) hervorstehen,
wobei das Prisma (22) eine erste Oberfläche (24) innerhalb des Gehäuses (10) und eine zweite Oberfläche (25) außerhalb des Gehäuses (10) umfasst und dafür eingerichtet ist, mit der Flüssigkeit in Kontakt zu sein,
**dadurch gekennzeichnet, dass** der Emitter (31) und der Empfänger (32) der Akustikeinheit (30) voneinander getrennt sind und sich die zweite Oberfläche (25) des Prismas (22) des Refraktometers (20) zwischen dem Emitter (31) und dem Empfänger (32) der Akustikeinheit (30) befindet.

2. Vorrichtung (1) nach Anspruch 1, einen Temperatursensor (40) umfassend, der in axialer Richtung aus der operativen Oberfläche (14) des Gehäuses (10) hervorsteht.

3. Vorrichtung (1) nach einem der vorhergehenden Ansprüche, eine elektronische Steuereinheit (50) umfassend, die für Folgendes konfiguriert ist:
• Empfangen eines ersten Signals von dem Refraktometer (20), das den Brechungsindex der Flüssigkeit angibt,
• Empfangen eines zweiten Signals von der Akustikeinheit (30), das die Schallausbreitungsgeschwindigkeit in der Flüssigkeit angibt,
• Bestimmen des Wertes der Konzentration mindestens eines ersten Bestandteils der Flüssigkeit in Abhängigkeit von dem ersten und dem zweiten Signal.

4. Vorrichtung (1) nach Anspruch 3, wobei die elektronische Steuereinheit (50) zum Bestimmen des Wertes der Konzentration eines zweiten Bestandteils der Flüssigkeit in Abhängigkeit von dem ersten und dem zweiten Signal konfiguriert ist.

5. Vorrichtung (1) nach einem der Ansprüche 3 oder 4, ein Anzeigeelement umfassend, das zum Anzeigen des Wertes der Konzentration des mindestens einen Bestandteils der Flüssigkeit konfiguriert ist.

6. Vorrichtung (1) nach einem der vorhergehenden Ansprüche, ein Energieversorgungselement umfassend, das in dem Gehäuse (10) enthalten ist und zum gleichzeitigen Versorgen des Refraktometers (20) und der Akustikeinheit (30) mit Energie konfiguriert ist.

7. Vorrichtung (1) nach einem der vorhergehenden Ansprüche, die mit Mitteln (13) zum Befestigen des Gehäuses (10) an einer Transportleitung für die Flüssigkeit versehen ist.

## Revendications

1. Dispositif (1) pour mesurer les paramètres physiques d'un liquide, comprenant un boîtier (10), à l'intérieur duquel sont placés :
• un réfractomètre (20) configuré pour mesurer une valeur de l'indice de réfraction du liquide, et
• une unité sonore (30) configurée pour mesurer une valeur de la vitesse de propagation du son dans le liquide,
dans lequel le boîtier (10) comprend une surface opérationnelle (14) conçue pour être en contact avec le liquide et dans lequel le réfractomètre (20) et l'unité sonore (30) sont associés à la dite surface opérationnelle (14) du boîtier (10),
dans lequel :
• le réfractomètre (20) comprend un prisme (22) faisant saillie de la surface opérationnelle (14) du boîtier (10) ; et
• l'unité sonore (30) comprend un émetteur (31) et un récepteur (32) d'un signal acoustique, qui fait saillie de la surface opérationnelle (14) du boîtier (10),
dans lequel le prisme (22) comprend une première surface (24) à l'intérieur du boîtier (10) et une seconde surface (25) à l'extérieur du boîtier (10) et conçue pour être en contact avec le liquide,
**caractérisé en ce que** l'émetteur (31) et le récepteur (32) de l'unité sonore (30) sont séparés l'un de l'autre, et la seconde surface (25) du prisme (22) du réfractomètre (20) est située entre l'émetteur (31) et le récepteur (32) de l'unité sonore (30).

2. Dispositif (1) selon la revendication 1, comprenant un capteur de température (40) faisant saillie dans une direction axiale de la surface opérationnelle (14) du boîtier (10).

3. Dispositif (1) selon l'une quelconque des revendications précédentes, comprenant une unité de commande électronique (50) configurée pour :
• recevoir un premier signal, envoyé par le réfractomètre (20), représentatif de l'indice de réfraction du liquide ;
• recevoir un second signal, envoyé par l'unité sonore (30), représentatif de la vitesse de propagation du son dans le liquide ;
• déterminer la valeur de la concentration d'au moins un premier composant du liquide en fonction du premier et du second signal.

4. Dispositif (1) selon la revendication 3, dans lequel l'unité de commande électronique (50) est configurée pour déterminer la valeur de la concentration d'un second composant du liquide en fonction du premier et du second signal.

5. Dispositif (1) selon l'une quelconque des revendications 3 ou 4, comprenant un élément d'affichage configuré pour afficher la valeur de la concentration d'au moins un composant du liquide.

6. Dispositif (1) selon l'une quelconque des revendications précédentes, comprenant un élément d'alimentation contenu dans le boîtier (10) et configuré pour alimenter de façon simultané le réfractomètre (20) et l'unité acoustique (30).

7. Dispositif (1) selon l'une quelconque des revendications précédentes, doté de moyens (13) pour fixer le boîtier (10) à un conduit d'acheminement pour le liquide.
